# EUROPÄISCHE PATENTANMELDUNG

(11) **EP 1 284 261 A1**
(43) Veröffentlichungstag der Anmeldung: **19.02.2003**
(21) Anmeldenummer: 02013874.9
(22) Anmeldetag: 22.06.2002
(51) Int. Cl.: C07D 239/54

(54) **Verfahren zur Herstellung von 4,6-Dihydroxypyrimidin**

(30) Priorität: 16.08.2001 DE 10140269
(71) Anmelder: Degussa AG, 40474 Düsseldorf (DE)
(72) Erfinder: Küver, Andreas, Dr., 53347 Alfter (DE); Hunds, Artur, 53121 Bonn (DE)

(57) **Zusammenfassung**

Die Erfindung betrifft ein Verfahren zur Herstellung von 4,6-Dihydroxypyrimidin durch Umsetzung von Malonsäurediester mit Formamid und Alkali-Alkoholat. Dabei wird der Malonsäureester allein oder gleichzeitig mit der Gesamtmenge oder einer Teilmenge des Formamids portionsweise oder kontinuierlich unter Temperaturkontrolle dem als Lösung oder Suspension in einem Alkohol allein oder zusammen mit der Gesamtmenge oder der restlichen Teilmenge des Formamids vorgelegten Alkali-Alkoholat zugefügt. Anschließend wird eine Temperatur von 102 bis < 120 °C für 10 bis < 60 Minuten eingestellt. Die Aufarbeitung nach der Haltezeit in dem genannten Temperaturbereich erfolgt dann in an sich bekannter Weise.

## Beschreibung

Die Erfindung betrifft ein Verfahren zur Herstellung von 4,6-Dihydroxypyrimidin (DHP), in seiner tautomeren Form auch 1-H-Pyrimidin-4,6-dion genannt, aus Malonsäurediester, Formamid und Alkali-Alkoholat. DHP ist ein wertvolles Zwischenprodukt für Wirkstoffsynthesen. So kann man aus 4,6-Dihydroxypyrimidin das entsprechende Dichlorpyrimidin herstellen, das seinerseits zu neuen, hochwirksamen Fungiziden verarbeitet werden kann (Europäische Patentanmeldungen 0 382 375, 0 393 861, 0 468 684 und 0 468 695).
Die meisten bekannten Verfahren zur Herstellung von 4,6-Dihydroxypyrimidin gehen von Malonsäurediamid aus und setzen dieses mit Ethylformiat um (siehe zum Beispiel R. Hull, J. Chem. Soc., 1951, 2214 und C. Hennart und E. Merlin, Bull. Soc. Chim., 1959, 741). Ebenso stellt die Reaktion von Malonsäureamid mit Formamid einen bekannten Zugang zu DHP dar (siehe D. J. Brown, J. Chem. Soc., 1956, 2312 - 2314 und A. Sömmer, DE-OS 12 00 308 beziehungsweise V. A. Zasonov, Khim.-Farm. Zh., Vol. 8, Nr. 12, 28 - 31). In der Arbeit von Zasonov wird das Malonsäureamid vor der Cyclokondensationsreaktion aus Malonsäureester und Ammoniak gebildet. Das Amid wird vor der weiteren Verwendung isoliert. Alle vom Malonamid ausgehenden Verfahren haben jedoch den gemeinsamen Nachteil, dass das Amid nicht kommerziell erhältlich ist und dass Stickstoffquellen in mehrstufigen Herstellungsprozessen für DHP nicht effizient genutzt werden, weil über die einzelnen Stufen Verluste auftreten. Diese Kritik erstreckt sich auch auf die Europäische Patentanmeldung EP-A-0 852 580 (Lonza AG), in der zwar ein eleganter Zugang zum Malondiamid beziehungsweise Malonmonoamidmonoester offengelegt wird, deren weiterer Umsetzung mit Formamid aber die oben genannten Nachteile anhaften.
Die Europäische Patentanmeldung EP-A-0 816 345 lehrt nun, dass sich DHP dergestalt darstellen lässt, dass man einstufig den Malonester, das Formamid und das Alkoholat unter erhöhter Temperatur und Eigendruck miteinander reagieren lässt, wobei sich DHP in guten Ausbeuten, sehr guten Raum-Zeit-Ausbeuten und hohen Nutzungsgraden der Stickstoffquelle Formamid bildet. Das in EP-A-0 816 345 beschriebene Verfahren stellt somit eine gegenüber dem vorherigen Stand der Technik deutliche Verbesserung dar. Insbesondere die integrierte Auflösung des intermediär erhaltenen DHP-Alkalisalzes und die erst in der letzten Stufe erfolgende Feststoffisolierung machen das Verfahren zusätzlich vorteilhaft.
Nachteilig ist an dem genannten Verfahren jedoch, dass man relativ lange Dosier- und Nachreaktionszeiten benötigt, so dass es im Sinne einer wirtschaftlichen Prozessführung wünschenswert wäre, diese Zeiten weiter zu verkürzen. Ein weiterer offensichtlicher Nachteil ist, dass die mit der kommerziell erhältlichen 30 %igen Lösung von Natriummethanolat in Methanol (NM30) erreichbaren Ausbeuten um bis zu 9 % unter denen liegen, die man mit höher konzentrierten Methanolatlösungen gemäß den Beispielen der EP-A-0 816 345 erreichen kann.

Es bestand daher die Aufgabe, ein Verfahren zur Herstellung von 4,6-Dihydroxypyrimidin bereitzustellen, das die oben beschriebenen Nachteile nicht aufweist.

Gegenstand der Erfindung ist ein Verfahren zur Herstellung von 4,6-Dihydroxypyrimidin (DHP), in seiner tautomeren Form auch 1-H-Pyrimidin-4,6-dion genannt, aus Malonsäurediester, Formamid und Alkali-Alkoholat, wobei überraschend gefunden wurde, dass man durch Halten der Temperatur nach Beendigung der Dosierung, die bei entsprechend guter Wärmeabfuhr in vorzugsweise 10 bis 50 Minuten, insbesondere in 10 bis 30 Minuten und ganz besonders bevorzugt in circa 20 Minuten durchgeführt werden kann, in einem Bereich von 102 bis < 120 °C dann gegenüber den obengenannten Verfahren eine signifikante Steigerung der Ausbeute erhält, wenn die Haltezeit 10 bis < 60 Minuten beträgt und genau auf die verwendete Temperatur abgestimmt wird. Weiterhin wurde überraschend ein sehr scharfer Zusammenhang zwischen Haltezeit, Temperatur, Dauer und erreichter Ausbeute gefunden. So wurde insbesondere bei vergleichender Betrachtung der Temperaturstufen 90 °C, 105 °C und 120 °C und einer Reaktionszeit von 30 Minuten ein nahezu umgekehrt-parabolischer Verlauf der Ausbeute mit einem Maximum bei circa 105 °C gefunden.

Bei der Produktion insbesondere von Wirkstoffen ist es üblich, dass bezüglich enthaltener Nebenkomponenten beziehungsweise deren maximal zulässiger Konzentrationen bestimmte Spezifikationen eingehalten werden müssen. Im vorliegenden Fall der Produktion von 4,6-DHP liefert ein bei einer Reaktionstemperatur von 90 °C und einer Reaktionszeit von 1 Stunde arbeitendes Verfahren in nur 82 %iger Ausbeute (siehe Vergleichsbeispiel 1) spezifikationsgerechte Ware, die dadurch gekennzeichnet ist, dass der durch potentiometrische Titration ermittelte Gehalt an DHP ≥ 96 % ist, und dass der durch eine festgelegte HPLC-Methode ermittelte Gehalt der Nebenkomponente 2,4,6-Trihydroxynicotinsäureamid durch eine bestimmte relative Peakfläche gegeben ist.

Das erfindungsgemäße Verfahren eliminiert die genannten Nachteile dergestalt, dass bei einer Temperatur von 102 bis < 120 °C, vorzugsweise 103 bis 115 °C und besonders bevorzugt bei 103 bis 107 °C und einer Haltezeit von 10 bis < 60 Minuten, vorzugsweise 20 bis 40 Minuten und besonders bevorzugt bei ca. 25 bis 35 Minuten mit dem Arbeitspunkt 30 Minuten ein Produkt in erhöhter Ausbeute erhalten wird, welches zusätzlich bezüglich der genannten Spezifikationen einwandfrei ist, und welches gegenüber dem Standardverfahren in einer deutlich verkürzten Gesamtcycluszeit anfällt (siehe Beispiel 1). Es wurde überraschend gefunden, dass die Zeit nach beendeter Dosierung (Haltezeit) in dem angegebenen Temperaturbereich zwingend einzuhalten ist.

Als Alkalialkoholate werden vorzugsweise Natrium- oder Kalium-Alkoholate, insbesondere Natriumalkoholate verwendet. Der alkoholische Rest im Alkoholat enthält 1 bis 4, vorzugsweise 1 bis 2, insbesondere 1 Kohlenstoffatom, vorzugsweise also die Methyl- oder Ethylgruppe. Ganz besonders bevorzugt ist die Verwendung von Natriummethanolat.

Als zusätzliche Bedingung ist zu berücksichtigen, dass der Gehalt an Cyanid, welcher sich durch Zersetzung von Formamid bilden kann, in der alkalischen Phase vor Fällung möglichst gering sein sollte, um eine Verschleppung von Cyanwasserstoff (HCN) in das Abwasser und/oder die Anlagenteile für die Fällung, Filtration und Trocknung zu vermeiden. Durch das oben geschilderte Verfahren wurde gefunden, dass bei Verwendung der relativ kurzen Haltezeit ein tolerabler Cyanidgehalt von nur [CN⁻] ≤ 5 ppm in der alkalischen Phase vor Filtration gefunden wird.

In weiteren Untersuchungen wurde beobachtet, dass man bei einer Durchführung des Verfahrens bei 120 °C / 20 Minuten zwar die genannten Erhöhungen der Ausbeute und Raum-Zeit-Ausbeute wiederum findet, dass aber die Spezifikation knapp verfehlt wird (siehe Vergleichsbeispiel 2). Insofern wurde gefunden, dass mit dem Arbeitsbereich 102 bis < 120 °C und 10 bis < 60 Minuten, insbesondere 103 - 107 °C und 25 - 35 Minuten und ganz besonders bevorzugt bei 105 °C und 30 Minuten gegenüber dem Standardverfahren eine überraschende Steigerung der Ausbeute und Reinheit, und damit auch der Produktivität erzielt werden kann.
Der genannte scharfe Zusammenhang zwischen Zeit und Temperatur wird noch deutlicher, wenn man Ergebnisse betrachtet, die mit einer Reaktionsführung bei 120 °C / 30 Minuten erzielt wurden (siehe Vergleichsbeispiel 3): hier wird die Standardausbeute unterschritten.

Es wurde zudem überraschend gefunden, dass sich die genannten Zusammenhänge zwischen Temperatur und Zeit mit großen Vorteilen auf Systeme übertragen lassen, in denen mit nicht handelsüblichen höherkonzentriertem Natriummethanolat (NM) gearbeitet wird. Insbesondere vorteilhaft erscheint die Verwendung einer 33 - 45 gew.-%igen, besonders vorteilhaft einer 33 bis 37 gew.-%igen, ganz besonders vorteilhaft die Verwendung einer 35 gew.-%igen Methanolatlösung -oder Suspension in Methanol (NM35), die sich durch Andestillieren der kommerziellen Ware NM30 im Reaktionsgefäß herstellen lässt. Hier ist aus dem Beispiel 2 und den Vergleichsbeispielen 4 und 5 zu ersehen, dass die Verwendung von NM35 nochmals eine Steigerung der Ausbeute nach sich zieht, wobei der beschriebene enge Temperatur-Zeit-Zusammenhang nach wie vor gilt. Eine analoge Aussage gilt für eine 40 gew.-% ige Methanolatlösung -oder Suspension, bei deren Verwendung sich eine weitere Ausbeutesteigerung ergibt, deren Verwendung aber nach einer Gesamtabwägung nicht unbedingt wirtschaftlicher zu sein scheint. Die Dosierung der Reaktanden kann in der Form erfolgen, dass der Malondiester allein oder gleichzeitig mit der Gesamtmenge oder einer Teilmenge des Formamids portionsweise oder kontinuierlich dem als Lösung oder Suspension in einem Alkohol allein oder zusammen mit der Gesamtmenge oder der restlichen Teilmenge des Formamids vorgelegten Alkali-Alkoholat zugefügt wird.

Die eigentliche Umsetzung des Malonsäurediesters mit dem Formamid erfolgt in an sich bekannter Weise im Bereich von üblicherweise 20 bis 80 °C (siehe Beispiele).

Der Malonsäurediester enthält vorzugsweise Alkylgruppen mit 1 bis 4 Kohlenstoffatomen.

Es wurde außerdem insbesondere überraschend gefunden, dass für die Zudosierung des Malonsäurediesters normalerweise 20 Minuten völlig ausreichen und dadurch zum Beispiel gegenüber dem in EP-A-0 816 345 beschrieben Verfahren ohne weitere Nachteile eine deutliche Verkürzung der Gesamtprozesszeit erzielt werden kann.
Nach beendeter Haltezeit erfolgt die Aufarbeitung des Reaktionsgemisches in an sich bekannter Weise, in dem üblicherweise auf Normaldruck entspannt, mit Wasser versetzt, der pH-Wert eingestellt und das ausgefallene Salz durch eine mechanische Trennoperation wie zum Beispiel Filtrieren, Dekantieren oder Zentrifugieren abgetrennt, gewaschen und getrocknet wird (siehe Beispiele).

Die folgenden Beispiele sollen die Erfindung weiter erläutern, jedoch nicht ihren Umfang begrenzen, wie er in den Patentansprüchen dargelegt ist.

### Beispiel 1

In einem 21-Drehrührautoklaven werden 3,3 mol Natriummethanolat in Methanol (NM30) sowie 2,25 mol Formamid vorgelegt. Das Gemisch wird auf 50 bis 55 °C erwärmt. Dann wird 1 mol Malonsäuredimethylester innerhalb von 20 Minuten zugepumpt, wobei sich die Mischung nicht über 65 °C erwärmt. Nach vollendeter Zugabe stellt man die Temperatur für 30 min auf 105 °C, entspannt dann den Autoklaven und spült mit Stickstoff. Danach wird das Reaktionsgemisch mit 460 ml vollentsalztem Wasser versetzt. Unter Kühlen werden anschließend unter pH-Kontrolle ca. 300 g wässrige 36 gew.-%ige Salzsäure zugetropft, wobei die Temperatur zwischen 20 und 25 °C gehalten wird. Das ausgefallene DHP wird abgesaugt und dreimal mit Wasser gewaschen. Nach dem Trocknen bei 70 ― 80 °C / 20 ― 30 mbar erhält man 94,83 g spezifikationsgerechtes DHP, entsprechend einer Ausbeute von 84,6 % d. Th.

### Beispiel 2

In einem 2l-Drehrührautoklaven werden 3,3 mol Natriummethanolat als NM35 sowie 2,25 mol Formamid vorgelegt. Das Gemisch wird auf 50 bis 55 °C erwärmt. Dann wird 1 mol Malonsäuredimethylester innerhalb von 20 Minuten zugepumpt, wobei sich die Mischung nicht über 65 °C erwärmt. Nach vollendeter Zugabe stellt man die Temperatur für 30 min auf 105 °C, entspannt dann den Autoklaven und spült mit Stickstoff. Das Gemisch wird wie in Beispiel 1 mit vollentsalztem Wasser versetzt und aufgearbeitet. Nach dem Trocknen bei 70 ― 80 °C / 20 ― 30 mbar erhält man 96,96 g spezifikationsgerechtes DHP, entsprechend einer Ausbeute von 86,5 % d. Th.

### Vergleichsbeispiel 1

Es wird wie in Beispiel 1 verfahren, jedoch stellt man die Temperatur nach beendeter Zugabe für 60 min auf 90 °C. Man erhält nur 91,35 g spezifikationsgerechtes DHP, entsprechend einer Ausbeute von 81,5 % d. Th.

### Vergleichsbeispiel 2

Es wird wie in Beispiel 1 verfahren, jedoch stellt man die Temperatur nach beendeter Zugabe für 20 min auf 120 °C. Man erhält 94,27 g nicht spezifikationsgerechtes DHP (Peakfläche im HPLC-Reinheitstest zu groß), entsprechend einer Ausbeute von 84,1 % d. Th.

### Vergleichsbeispiel 3

Es wird wie in Beispiel 1 verfahren, jedoch stellt man die Temperatur nach beendeter Zugabe für 30 min auf 120 °C. Man erhält 90,23 g spezifikationsgerechtes DHP. Die Ausbeute beträgt jedoch nur 80,5 % d. Th.

### Vergleichsbeispiel 4

Es wird wie in Beispiel 2 verfahren, jedoch stellt man die Temperatur nach beendeter Zugabe für 60 min auf 90 °C. Man erhält 92,92 g spezifikationsgerechtes DHP. Die Ausbeute beträgt jedoch nur 82,9 % d. Th.

### Vergleichsbeispiel 5

Es wird wie in Beispiel 2 verfahren, jedoch stellt man die Temperatur nach beendeter Zugabe für 30 min auf 120 °C. Man erhält 95,95 g nicht spezifikationsgerechtes DHP, entsprechend einer Ausbeute von 85,6 % d. Th.

## Patentansprüche

1. Verfahren zur Herstellung von 4,6-Dihydroxypyrimidin durch Umsetzung von Malonsäurediester mit Formamid und Alkali-Alkoholat,
**dadurch gekennzeichnet,**
**dass** der Malondiester allein oder gleichzeitig mit der Gesamtmenge oder einer Teilmenge des Formamids portionsweise oder kontinuierlich dem als Lösung oder Suspension in einem Alkohol allein oder zusammen mit der Gesamtmenge oder der restlichen Teilmenge des Formamids vorgelegten Alkali-Alkoholat zugefügt wird und nach beendeter Dosierung die Temperatur des Reaktionsgemisches auf 102 bis <120 °C eingestellt und 10 bis < 60 Minuten gehalten wird.

2. Verfahren nach Anspruch 1,
**dadurch gekennzeichnet,**
**dass** die Dosierung innerhalb von 10 bis 50 Minuten durchgeführt wird.

3. Verfahren nach Anspruch 1,
**dadurch gekennzeichnet,**
**dass** die Temperatur nach der Dosierung auf 103 bis 115 °C eingestellt wird.

4. Verfahren nach Anspruch 1,
**dadurch gekennzeichnet,**
**dass** die Temperatur nach der Dosierung auf 103 bis 107 °C eingestellt wird.

5. Verfahren nach einem der Ansprüche 1 bis 4,
**dadurch gekennzeichnet,**
**dass** die Temperatur des Reaktionsgemisches nach der Dosierung 20 bis 40 Minuten gehalten wird.

6. Verfahren nach einem der vorhergehenden Ansprüche,
**dadurch gekennzeichnet,**
**dass** der Malonsäurediester Alkylgruppen mit 1 bis 4 Kohlenstoffatomen enthält.

7. Verfahren nach einem der vorherhehenden Ansprüche,
**dadurch gekennzeichnet,**
**dass** das Alkoholat einen aliphatischen Rest mit 1 bis 4 Kohlenstoffatomen enthält.

8. Verfahren nach Anspruch 7,
**dadurch gekennzeichnet,**
**dass** das Alkoholat als aliphatischen Rest die Methyl- oder Ethylgruppe enthält.

9. Verfahren nach einem der vorhergehenden Ansprüche,
**dadurch gekennzeichnet,**
**dass** als Alkoholat Natriummethanolat eingesetzt wird.

10. Verfahren nach Anspruch 9,
**dadurch gekennzeichnet,**
**dass** das Natriummethanolat als 33 bis 45 gew.-%ige Lösung oder Suspension in Methanol eingesetzt wird.

11. Verfahren nach mindestens einem der vorhergehenden Ansprüche,
**dadurch gekennzeichnet,**
**dass** für die Reaktion eine 33 bis 37 gew.-%ige Natriummethanolat-lösung oder ―suspension in Methanol eingesetzt wird, die Temperatur nach Beendigung der Dosierung auf 103 ― 107 °C eingestellt wird und diese Temperatur 25 ― 35 Minuten gehalten wird.
